(19) Europäisches Patentamt
European Patent Office
Office européen des brevets

(11) Publication number: 0 260 127 A2

# (12) EUROPEAN PATENT APPLICATION

(21) Application number: 87307978.4

(22) Date of filing: 09.09.87

(51) Int. Cl.[4]: C 07 D 473/04
A 61 K 31/52

(30) Priority: 11.09.86 GB 8621870

(43) Date of publication of application:
16.03.88 Bulletin 88/11

(84) Designated Contracting States:
BE CH DE FR GB IT LI NL

(71) Applicant: BEECHAM - WUELFING GmbH & Co. KG
Stresemannallee 6 P.O. Box 25
D-4040 Neuss (DE)

BEECHAM GROUP PLC
Beecham House Great West Road
Brentford Middlesex TW8 9BD (GB)

(72) Inventor: Nicholson, Charles David
Beecham-Wülfing Betheiner Landstrasse
D-3212 Gronau (DE)

Göring, Joachim
Beecham-Wülfing Betheiner Landstrasse
D-3212 Gronau (DE)

Morgan, Brian
Beecham Pharmaceuticals Brockham Park
Betchworth Surrey, RH3 7AJ (GB)

Arch, Jonathan Robert Sanders
Beecham Pharmaceuticals Yew Tree Bottom Road
Epsom Surrey, KT18 5XQ (GB)

(74) Representative: Valentine, Jill Barbara et al
Beecham Pharmaceuticals Patents & Trade Marks Dept.
Great Burgh Yew Tree Bottom Road
Epsom Surrey KT18 5XQ (GB)

The title of the invention has been amended (Guidelines for Examination in the EPO, A-III, 7.3).

## (54) Xanthine derivatives, their preparation and pharmaceutical compositions containing them.

(57) A compound of formula (I):

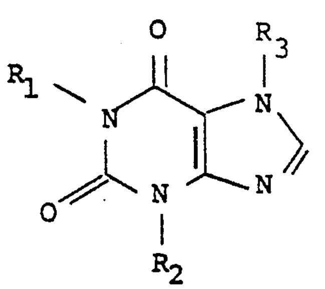

(I)

or a pharmaceutically acceptable salt thereof, wherein $R_1$ and $R_2$ are independently n-butyl, $-(CH_2)_2CHOHCH_3$, $-(CH_2)_2CHOHCH_2OH$, $-(CH_2)_2COCH_3$ or $-(CH_2)_2C(OR_4)(OR_5)CH_3$ where $R_4$ and $R_5$ are each $C_{1-4}$ alkyl or together are $C_{2-4}$ polymethylene, provided that $R_1$ and $R_2$ are not both n-butyl, and $R_3$ is $-CH_2CHOHCH_3$, $-CH_2COCH_3$ or $-CH_2C(OR_6)(OR_7)CH_3$ where $R_6$ and $R_7$ are each $C_{1-4}$ alkyl or together are $C_{2-4}$ polymethylene.

EP 0 260 127 A2

**Description**

ACTIVE COMPOUNDS

This invention relates to compounds having pharmacological activity, to a process for their preparation and their use as pharmaceuticals.

According to the present invention there is provided a compound of formula (I):

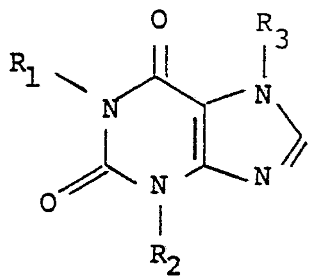

(I)

or a pharmaceutically acceptable salt thereof, wherein $R_1$ and $R_2$ are independently n-butyl, $-(CH_2)_2CHOHCH_3$, $-(CH_2)_2CHOHCH_2OH$, $-(CH_2)_2COCH_3$ or $-(CH_2)_2C(OR_4)(OR_5)CH_3$ where $R_4$ and $R_5$ are each $C_{1-4}$ alkyl or together are $C_{2-4}$ polymethylene, provided that $R_1$ and $R_2$ are not both n-butyl, and $R_3$ is $-CH_2CHOHCH_3$, $-CH_2COCH_3$ or $-CH_2C(OR_6)(OR_7)CH_3$ where $R_6$ and $R_7$ are each $C_{1-4}$ alkyl or together are $C_{2-4}$ polymethylene.

The compounds of the present invention have a protective effect against the consequences of cerebral metabolic inhibition. The compounds of the present invention also improve data acquisition or retrieval following transient forebrain ischaemia. The compounds are therefore useful in the treatment of cerebral vascular and neuronal degenerative disorders associated with learning, memory and cognitive dysfunctions including cerebral senility, multi-infarct dementia and senile dementia of the Alzheimer type.

The compounds of the present invention are also active in increasing the oxygen tension in ischaemic skeletal muscle. This property reflects an increase in the nutritional blood flow through ischaemic skeletal muscle which in turn indicates that the compounds of the invention are of potential use as agents for the treatment of peripheral vascular disease such as intermittent claudication.

The compounds of the present invention also act as phosphodiesterase inhibitors and elevate cyclic AMP levels and are therefore of potential use in the treatment of proliferative skin disease in human or non-human mammals.

As used herein the expression 'proliferative skin diseases' means benign and malignant proliferative skin diseases which are characterized by accelerated cell division in the epidermis, dermis or appendages thereto, associated with incomplete tissue differentiation. Such diseases include: psoriasis, atopic dermatitis, non-specific dermatitis, primary irritant contact dermatitis, allergic contact dermatitis, basal and squamous cell carcinomas of the skin, lamellar ichthyosis, epidermolytic hyperkeratosis, premalignant sun induced keratosis, non-malignant keratosis, acne, and seborrheic dermatitis in humans and atopic dermatitis and mange in domesticated animals.

The compounds of formula (I) can form acid addition salts with acids, such as the conventional pharmaceutically acceptable acids, for example hydrochloric, hydrobromic, phosphoric, acetic, fumaric, salicylic, citric, lactic, mandelic, tartaric, oxalic and methanesulphonic.

Where compounds of formula (I) can exist in more than one stereoisomeric form, the invention extends to each of these forms and to mixtures thereof.

The compounds of the invention are preferably in pharmaceutically acceptable form. By pharmaceutically acceptable form is meant, inter alia, of a pharmaceutically acceptable level of purity excluding normal pharmaceutical additives such as diluents and carriers, and including no material considered toxic at normal dosage levels. A pharmaceutically acceptable level of purity will generally be at least 50% excluding normal pharmaceutical additives, preferably 75%, more preferably 90% and still more preferably 95%.

$R_1$ and $R_2$ are preferably selected from n-butyl,

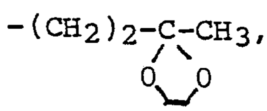

$-(CH_2)_2CHOHCH_2OH$, $-(CH_2)_2CHOHCH_3$ and $-(CH_2)_2COCH_3$.

$R_3$ is preferably selected from $-CH_2CHOHCH_3$, $-CH_2COCH_3$ and

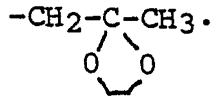

The invention further provides a process for the preparation of a compound of formula (I), or a pharmaceutically acceptable salt thereof, which process comprises reacting xanthine in which one of the 1 and 3 positions is substituted by $R_1'$, or $R_2'$, respectively, sequentially and in any appropriate order with compounds $R_3'X_3$ and $R_1'X_1$ or $R_2'$, as appropriate, in which $R_1'$, $R_2'$ and $R_3'$ are $R_1$, $R_2$ and $R_3$ respectively, or groups convertible thereto and $X_1$, $X_2$ and $X_3$ are leaving groups, optionally with protection of xanthine nitrogen atoms, and at any appropriate stage, as necessary, converting $R_1'$, $R_2'$ and $R_3'$ to $R_1$, $R_2$ and $R_3$, interconverting $R_1$, $R_2$ and $R_3$ and optionally forming a pharmaceutically acceptable salt of the resulting compound of formula (I).

Suitable examples of leaving groups $X_1$, $X_2$ and $X_3$ include halo such as chloro and bromo.

Suitable examples of groups $R_1'$, $R_2'$ and $R_3'$ convertible to $R_1$, $R_2$ and $R_3$ include protecting groups such as benzyl, alkenyl groups, ketals of oxoalkyl groups and, where $R_1$ or $R_2$ is 3,4-dihydroxybutyl, a ketal formed with a suitable ketone such as acetone.

The reaction of xanthine with compounds $R_1'X_1$, $R_2'X_2$ or $R_3'X_3$ is a nucleophilic substitution which may be carried out under conventional conditions, depending upon the leaving group $X_1$, $X_2$ or $X_3$. For example, when the leaving group is halo such as chloro or bromo, the reaction may be performed under basic conditions e.g. in the presence of an alkali metal carbonate such as potassium carbonate, in an inert polar solvent such as dimethylformamide, or with sodium in ethanol, at ambient to elevated temperature.

Protection of xanthine nitrogen atoms may be carried out at any appropriate stage in the process. Suitable protecting groups include benzyl, which may be introduced by reaction of the xanthine with a benzyl halide, such as the chloride or bromide. Suitable conditions for the nucleophilic substitution are those mentioned above for the reaction with compounds $R_1'X_1$, $R_2'X_2$ and $R_3'X_3$.

Protecting groups may be removed conventionally at an appropriate point in the procedure. Benzyl protecting groups may be removed by any convenient process, such as by catalytic hydrogenation over a suitable catalyst such as palladium on activated charcoal, in a suitable solvent such as ethanol at elevated temperature, or by treatment with anhydrous aluminium chloride in dry benzene at ambient temperature.

Appropriate alkenyl groups may be converted to the corresponding hydroxyalkyl groups by conventional acid hydrolysis.

Alternatively, alkenyl groups may be converted to the corresponding dihydroxy groups conventionally, for example by oxidation to the epoxide which may be opened hydrolytically, or by direct oxidation. Suitable oxidising agents include potassium permanganate, t-butyl hydrogen peroxide, osmium tetroxide and silver benzoate, and epoxide opening may conveniently be effected by treatment with aqueous acid or base.

Ketals of oxoalkyl groups or 3,4-dihydroxybutyl groups may be converted to oxoalkyl groups or 3,4-dihydroxybutyl groups, respectively, by conventional acid hydrolysis.

Interconversion of groups $R_1$, $R_2$ and $R_3$ may be carried out conventionally. Thus, oxoalkyl groups may converted to ketal groups within formula (I) by treatment with the appropriate alcohol in an inert solvent such as xylene, or the alcohol acting as the solvent, in the presence of an acid catalyst such as p-toluenesulphonic acid at elevated temperature. Ketal groups may be converted to oxoalkyl groups by conventional acid hydrolysis. Oxoalkyl groups may be converted to hydroxyalkyl groups by reduction with a suitable reducing agent such as sodium borohydride at ambient temperature in a suitable solvent such as methanol.

Pharmaceutically acceptable salts of the compounds of formula (I) may be formed conventionally by reaction with the appropriate acid such as described above under formula (I).

In one particular aspect, the process according to the invention comprises the reduction of a compound of formula (II):

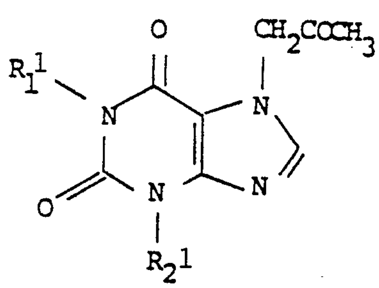

(II)

in which one of $R_1{}^1$ and $R_2{}^1$ is n-butyl and the other is $(CH_2)_2COCH_3$, optionally followed by the formation of a

pharmaceutically acceptable salt.

The reduction of the compound of formula (II) may be carried out under conventional conditions as described above for the conversion of oxoalkyl to hydroxylalkyl groups.

Compounds of formula (II) may be prepared by the reaction of a compound of formula (III):

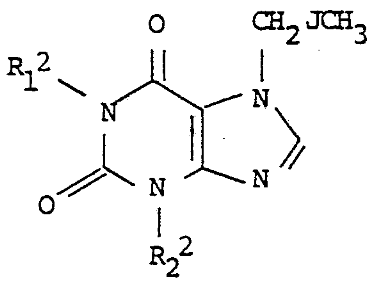

(III)

wherein one of $R_1^2$ and $R_2^2$ is hydrogen and the other is n-butyl, and J is an optionally protected carbonyl group, with a compound of formula (IV):

$Q(CH_2)_2COCH_3$ (IV)

where Q is a leaving group, followed as necessary by the deprotection of a protected carbonyl group J.

The reaction between the compounds of formulae (III) and (IV) may be carried out under conventional conditions for nucleophilic substitution as described above, for example under basic conditions e.g. in the presence of an alkali metal carbonate such as potassium carbonate, in an inert polar solvent such as dimethylformamide at elevated temperature.

When J is a protected carbonyl group such as a ketal thereof e.g. the 1,3-dioxolane, the deprotection step is carried out by conventional acid hydrolysis.

Compounds of formula (III) may be prepared by reacting a compound of formula (V):

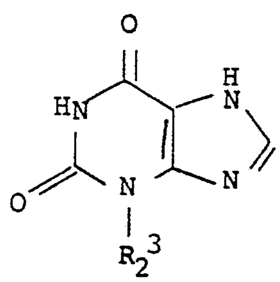

(V)

wherein $R_2^3$ is n-butyl or an N-protecting group, with a compound of formula (VI):

$XCH_2COCH_3$ (VI)

where X is a leaving group, followed as necessary by the removal of an N-protecting group and the protection of the carbonyl group in $-CH_2COCH_3$.

The reaction between the compounds of formulae (V) and (VI) may be carried out under conventional conditions for nucleophilic substitution as described above, for example with sodium in ethanol at ambient or slightly elevated temperature.

$R_3^3$ N-protecting groups are conventional groups such as benzyl which may be removed by conventional hydrogenation e.g. over a catalyst such as palladium.

The protection of the carbonyl group in $-CH_2COCH_3$ may be carried out conventionally depending upon the choice of protecting group. For example, ketalisation may be performed with the appropriate alcohol such as ethylene glycol under acid conditions e.g. in the presence of p-toluenesulphonic acid in an inert solvent such as xylene.

The 1 or 3 substituted xanthine starting material, such as a compound of formula (V) may be prepared conventionally, for example using the method of H.Bredereck and A. Edenhofer, Chem. Berichte 88, 1306-1312 (1955) from the corresponding substituted 6-aminouracils which may themselves be prepared by the method of V. Papesch and E. F. Schröder, J. Org. Chem. 16, 1879-90 (1951), or Yozo Ohtsuka, Bull. Chem. Soc. Jap. 1973, 46(2), 506-9.

The invention also provides a pharmaceutical composition comprising a compound of formula (I) or a pharmaceutically acceptable salt thereof and a pharmaceutically acceptable carrier.

The compositions may be in the form of tablets, capsules, powders, granules, lozenges, suppositories,

reconstitutable powders, or liquid preparations such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling, tabletting or the like. Repeated blending operations may be used to distribute the active agent throughout those compositions employing large quantities of fillers.

Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily ester such as esters of glycerine, propylene glycol, or ethyl alcohol; preservatives, for example methyl or propyl p-hydroxybenzoate or sorbic acid; and if desired conventional flavouring or colour agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing.

Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10-60% by weight, of the active material, depending on the method of administration.

Compounds of formula (I) may also be administered as a topical formulation in combination with conventional topical excipients.

Topical formulations may be presented as, for instance, ointments, creams of lotions, impregnated dressings, gels, gel sticks, spray and aerosols, and may contain appropriate conventional additives such as preservatives, solvents to assist drug penetration and emollients in ointments and creams. The formulations may contain compatible conventional carriers, such as cream or ointment bases and ethanol or oleyl alcohol for lotions.

Suitable cream, lotion, gel, stick, ointment, spray or aerosol formulations that may be used for compounds of formula (I) are conventional formulations well known in the art, for example, as described in standard text books of pharmaceutics and cosmetics, such as Harry's Cosmeticology published by Leonard Hill Books, Remington's Pharmaceutical Sciences, and the British and US Pharmacopoeias.

Suitably, the compound of formula (I) will comprise from about 0.5 to 20% by weight of the formulation, favourably from about 1 to 10%, for example 2 to 5%.

The invention also provides a method of treatment of cerebrovascular disorders and/or disorders associated with cerebral senility in mammals including humans, which comprises administering to the sufferer an effective amount of a compound of formula (I) or a pharmaceutically acceptable salt thereof.

The invention further provides a method of treatment in mammals including humans of cerebral vascular and neuronal degenerative disorders associated with learning, memory and cognitive dysfunctions including cerebral senility, multi-infarct dementia and senile dementia of the Alzheimer type, which comprises administering to the sufferer an effective amount of a compound of formula (I) or a pharmaceutically acceptable salt thereof.

The invention further provides a method for the treatment of peripheral vascular disease in mammals including humans, which comprises administering to the sufferer an effective amount of a compound of formula (I) or a pharmaceutically acceptable salt thereof.

In another aspect, the present invention provides a method for the treatment of proliferative skin disease in mammals including humans which comprises administering to the mammal in need of such treatment an effective amount of a compound of formula (I) or a pharmaceutically acceptable salt thereof.

The dose of the compound used in the treatment of such disorders will vary in the usual way with the seriousness of the disorders, the weight of the sufferer, and the relative efficacy of the compound. However, as a general guide suitable unit doses may be 0.5 to 1000mg. for example 0.5 to 200mg; and such unit doses

may be administered more than once a day, for example two or three times a day, so that the total daily dosage for a 70kg adult is in the range of about 1 to 1000 mg, that is in the range of about 0.02 to 20 mg/kg/day; and such therapy may extend for a number of weeks or months.

At the above described dosage range, no toxicological effects are indicated for the compounds of the present invention.

In a further aspect the invention provides a compound of formula (I) for use as an active therapeutic substance.

The invention further provides a compound of formula (I) or a pharmaceutically acceptable salt thereof, for use in the treatment cerebral vascular and neuronal degenerative disorders associated with learning, memory and cognitive dysfunctions including cerebral senility, multi-infarct dementia and senile dementia of the Alzheimer type and/or peripheral vascular disease and/or proliferative skin diseases.

In another aspect, the invention provides the use of a compound of formula (I) or a pharmaceutically acceptable salt thereof for the preparation of a medicament for the treatment of cerebral vascular and neuronal degenerative disorders associated with learning, memory and cognitive dysfunctions including cerebral senility, multi-infarct dementia and senile dementia of the Alzheimer type and/or peripheral vascular disease and/or proliferative skin diseases.

The following examples illustrate the invention and the following descriptions illustrate the preparation of intermediates thereto.

Description 1

3-n-Butyl-1-7-(2-oxopropyl)xanthine (D1)

26.0 g Pulverized 3-n-butylxanthine(Bull. Chem. Soc. Jap. 1973, 46(2), 506-9, but prepared by the methods of Chem. Berichte 88, 1306-1312 (1955) and J. Org. Chem. 16, 1879-90 (1951))was added, stepwise, to a freshly prepared solution of 2.86 g sodium in 125 ml absolute ethanol, to give a clear solution. At a temperature of ca. 30°C, a solution of 17.3 g chloracetone in 125 ml absolute ethanol was then added dropwise, with stirring. After two hours, the precipitated sodium chloride, together with the crude 3-n-butyl-7-(2-oxopropyl)xanthine, was filtered off by suction and the residue washed with absolute ethanol.

To remove the sodium chloride from this mixture, it was suspended in 700 ml of water, with stirring, for 30 min. The suspension was then filtered off by suction and the residue dissolved in dilute aqueous sodium hydroxide solution.

To this alkaline solution, dilute hydrochloric acid was added dropwise with stirring to ca. pH 10. The precipitated 3-n-butyl-7-(2-oxopropyl)xanthine was filtered off under suction, and the residue was recrystallised from ethanol to yield the product as a white powder.

Melting point: 218°C
Yield: 10.1g = 30.6 % of theory.

Elemental analysis:

| | Calculated | Found |
|---|---|---|
| C | 54.54 | 54.58 |
| H | 6.11 | 6.11 |
| N | 21.19 | 21.10 |
| O | 18.16 | 18.19 |

Description 2

3-Benzyl-7-(2-oxopropyl)xanthine (D2)

Compound D2 was prepared by an analgous procedure to that of Description 1 from 3-benzylxanthin (Bull. Chem. Soc. Jap. 1973, 46(2), 506-9, but prepared by the methods of Chem. Berichte 88, 1306-1312 (1955) and J. Org. Chem. 16, 1879-90 (1951)).

Melting point: 252°C.

Description 3

2-Methyl-2-[(3-n-butylxanthin-7-yl)methyl]-1,3-dioxolane (D3)

3-n-Butyl-7-(2-oxopropyl)xanthine (10.5g)(D1), ethylene glycol (5.5 ml), xylene (65 ml) and p-toluenesulphonic acid (0.05 g ) were treated for several days under reflux.

The reaction water was removed by a water separator. After cooling at room temperature the precipitated compound was filtered off by suction, washed with xylene, dried and recrystallised from ethanol to yield 9.3 g of solid, m.p. 179°C.

Description 4

1-n-Butyl-3-benzyl-7-(2-oxopropyl)xanthine (D4)

3-Benzyl-7-(2-oxopropyl)xanthine (D2) (4.5g), potassium carbonate (2.1g) and dimethylformamide (25ml) was heated to 120°C to give a clear solution.

With stirring, 1-brombutane (2.05g) was added dropwise at this temperature. Within a few minutes the reaction was complete and potassium bromide precipitated.

After cooling at room temperature the potassium bromide was removed by suction.

From the filtrate the dimethylformamide was removed by distillation under reduced pressure, the residue extracted with chloroform, the chloroform phase washed twice with 1N sodium hydroxide, twice with water and then dried over sodium sulphate, filtered and the resulting filtrate distilled under reduced pressure to remove the chloroform.

The residue was recrystallised from methanol to yield 2.2g of a solid.

Melting point: 130°C

Description 5

1-n-Butyl-7-(2-oxopropyl)xanthine

1-n-Butyl-3-benzyl-7-(2-oxopropyl)xanthine (D4) (3.5 g) was dissolved in absolute ethanol (100ml) at 60°C then palladium on activated charcoal [10% Pd] (0.18 g) was added and hydrogen introduced under normal pressure. The course of reaction was controlled by tlc. After four hours the reaction was nearly complete and the reaction mixture was cooled to room temperature, filtered, and the filtrate concentrated to dryness under reduced pressure.

The residue was treated with chloroform and then the chloroform phase extracted with 1N sodium hydroxide solution. The sodium hydroxide phase was separated and filtered, diluted hydrochloric acid was added dropwise with stirring to this filtrate and the precipitated compound was obtained by suction and washed with water.

This compound was recrystallised from methanol to yield 0.7 of a solid.

Melting point 214°C

Alternative route

To anhydrous aluminium chloride (36.1g, 0.27mol) was added a solution of 1-n-butyl-3-benzyl-7-(2-oxooxopropyl)xanthine (D4) (24.0g, 0.068mol) in dry benzene (400 ml). The mixture was stirred at room temperature for 3h (controlled by tlc).

After this time ice/water was added and the reaction mixture was allowed to stand overnight.

From this biphase reaction mixture, 1-n-butyl-7-(2-oxopropyl)xanthine precipitated and was isolated by suction, washed with water and recrystallised from methanol.

Yield: 12.4g = 68.9%
Melting point: 215°C.

Description 6

2-Methyl-2-[(1-n-butylxanthin-7-yl)methyl]-1,3-dioxolane (D6)

Compound D6 was prepared from compound D5 by an analogous procedure to that of Description 3.

Melting point: 131°C

Description 7

3-n-Butyl-7-(2-hydroxypropyl)xanthine (D7)

Using an analogous procedure to that of Example 5, compound D7 was prepared from compound D1.

Description 8

1-n-Butyl-7-(2-hydroxypropyl)xanthine (D8)

Using an analogous procedure to that of Example 5, compound D8 was prepared from compound D5.

Description 9

1-(3-Hydroxybutyl)-3-benzyl-7-(2-oxopropyl)xanthine (D9)

Using an analogous procedure to that of Example 7, compound D9 was prepared from compound D2.

Melting point: 142°C.

Description 10

1-(3-Hydroxybutyl)-7-(2-oxopropyl)xanthine (D10)

Using an analogous procedure to that of Description 5 (Alternative Procedure), compound D10 was prepared from compound D9.

Melting point: 234°C.

Description 11

2-Methyl-2-[(3-benzylxanthin-7-yl)methyl]-1,3-dioxolane (D11)

Compound D11 was prepared from compound D2 by an analogous procedure to that of Description 3.

Melting point: 202°C

Description 12

2-Methyl-2-[(1-(3-oxobutyl)-3-benzylxanthin-7-yl)methyl]-1,3-dioxolane (D12)

Compound D12 was prepared from compound D11 by an analogous procedure to that of Example 1.

Melting point: oil.

Description 13

1-(3-Oxobutyl)-3-benzyl-7-(2-oxopropyl)xanthine (D13)

Compound D13 was prepared from compound D12 (as unpurified raw material) by an analogous procedure to that of Example 3.

Melting point: 158°C.

Description 14

1-(3-Oxobutyl)-7-(2-oxopropyl)xanthine (D14)

Compound D14 was prepared from compound D13 by an analogous procedure to that of Description 5 (alternative route).

Melting point: 205°C

Description 15

1-(4-[2-Methyl-1,3-dioxolanyl-(2)]butyl)-7-(3-[2-methyl-1,3-dioxolanyl-(2)]propyl)xanthine (D15)

Compound D15 was prepared from compound D14 by an analogous procedure to that of Description 3.

Melting point 178°C

Description 16

1-(3,4-Isopropylidenedioxybutyl)-3-n-butyl-7-(2-oxopropyl)xanthine (D16)

Compound (12.3g), finely divided potassium carbonate (6.9g), dimethyl formamide (85ml) and 5-(β-bromoethyl)-2,2-dimethyl-1,3-dioxolane (prepared as described in British Patent No. 1036085) were heated continuously up to 100°C with stirring, then left to stand overnight at room temperature.

On the next day the reaction was completed by further heating at 100°C for 1 hour with stirring, then coolingto room temperature.

The precipitated potassium bromide was removed by suction and the reaction mixture was concentrated under reduced pressure, the residue extracted with methylene chloride, the methylene chloride phase washed

with 1N sodium hydroxide, twice with water and then dried over sodium sulphate, filtered and the resulting filtrate distilled under reduced pressure to remove the methylene chloride.

The residue was recrystallised from diethylether to yield 12.1g product.

The structures of the compounds of Descriptions 1 to 6 and 9 to 16 were confirmed by NMR spectroscopy.

Example 1

2-Methyl-2-[(1-(3-oxobutyl)-3-n-butylxanthin-7-yl) methyl]-1,3-dioxolane (E1).

2-Methyl-2-[(3-n-butylxanthin-7-yl)methyl]-1, 3-dioxolane (D3) (3.6 g), potassium carbonate (3.15 g) and dimethylformamide (38 ml) were stirred together then heated to 120°C.

At this temperature 4-chlorobutan-2-one(3.6g) was added dropwise. Within 15 minutes the reaction was completed. After cooling to room temperature, the precipitated potassium chloride was removed by suction. From the filtrate the dimethylformamide was removed by distillation under reduced pressure and the residue extracted with chloroform, the chloroform phase washed twice with 1N sodium hydroxide, twice with water and then dried over sodium sulphate, filtered and the resulting filtrate distilled under reduced pressure to remove chloroform.

The residue was recrystallised from ether to yield 5.4g of solid.

Melting point: 87°C

Example 2

2-Methyl-2-[(1-n-butyl-3-(3-oxobutyl)xanthin-7-yl) methyl]-1,3-dioxolane (E2)

Compound E2 was prepared from compound D6 by an analogous procedure to that of Example 1.

Melting point: 74°C

Example 3

1-(3-Oxobutyl)-3-n-butyl-7-(2-oxopropyl)xanthine (E3)

2-Methyl-2-[(1-(3-oxobutyl)-3-n-butyl)xanthin-7-yl) methyl]-1,3 dioxolane (E1) (1 g), ethanol (10 ml), water (5 ml) and concentrated hydrochloric acid (1 ml) were heated for 6 hours under reflux with stirring. After cooling the solvents were removed *in vacuo*. The residue was extracted with water, the chloroform phase washed with water, then dried over sodium sulphate, filtered and the chloroform removed *in vacuo*.

The residue was recrystallised from ethanol to yield 0.55 g of a solid.

Melting point: 115°C

Example 4

1-n-Butyl-3-(3-oxobutyl)-7-(2-oxopropyl)xanthine (E4)

Compound E4 was prepared from compound E2 by a procedure analogous to that of Example 3.

Melting point: 111°C

Example 5

1-(3-Hydroxybutyl)-3-n-butyl-7-(2-hydroxypropyl) xanthine (E5)

1-(3-Oxobutyl)-3-n-butyl-7-(2-oxopropyl)xanthine (E3) (0.3g) was completely dissolved in methanol, and sodium borohydride (0.2g) was added in portions at room temperature. After some minutes the reaction was complete (controlled by tlc). The reaction mixture was diluted with water and treated with dilute hydrochloric acid and extracted with chloroform and the chloroform phase washed with water, dried over sodium sulphate, filtered and the chloroform removed *in vacuo*.

The residue solidified overnight and was recrystallised from ether to yield 0.15g of a solid.

Melting point: 105°C

Elemental analysis:

| | Calculated | Found |
|---|---|---|
| C | 56.79 | 57.16 |
| H | 7.74 | 7.73 |
| N | 16.56 | 16.70 |
| O | 18.91 | 18.66 |

Example 6

1-n-Butyl-3-(3-hydroxybutyl)-7-(2-hydroxypropyl)xanthine (E6)

Compund E6 was prepared from E4 by a procedure analogous to that of Example 5.

Melting point: 80-85°C.

Elemental analysis:

| | Calculated | Found |
|---|---|---|
| C | 56.79 | 57.08 |
| H | 7.74 | 7.78 |
| N | 16.56 | 16.49 |
| O | 18.91 | 18.84 |

Example 7

1-(3-Hydroxybutyl)-3-n-butyl-7-(2-oxopropyl)xanthine (E7)

3-n-Butyl-7-(2-oxopropyl)xanthine (D1)(5.3g), finely divided potassium carbonate (2.8g) and dimethylformamide (33ml) was heated to 120°C.

After 30 minutes at this temperature 4-chlorobutan-2-ol was added dropwise with stirring.

After 2h the reaction was complete (controlled by tlc) and the reaction mixture was cooled to room temperature.

The precipitated potassium chloride was removed by suction and the filtrate was concentrated under reduced pressure, the residue extracted with chloroform, the chloroform phase washed with 1N sodium hydroxide, twice with water and then dried over sodium sulphate, filtered and the resulting filtrate distilled under reduced pressure to remove the chloroform.

The residue was recrystallised from methanol and charcoal to yield 2.4g solid.

Melting point: 99°C.

Elemental analysis.

| | Calculated | Found |
|---|---|---|
| C | 57.13 | 57.06 |
| H | 7.19 | 7.20 |
| N | 16.66 | 16.78 |
| O | 19.02 | 19.00 |

Example 8

1-(3-Oxobutyl)-3-n-butyl-7-(2-hydroxypropyl) xanthine (E8)

Compound E8 was prepared from compound D7 (as unpurified raw material) by an analogous procedure to that of Example 1.

Melting point: 77 to 79°C.

Elemental analysis

| | Calculated | Found |
|---|---|---|
| C | 57.13 | 57.10 |
| H | 7.19 | 7.23 |
| N | 16.66 | 16.70 |
| O | 19.02 | 19.01 |

Example 9

1-n-Butyl-3-(3-hydroxybutyl)-7-(2-oxopropyl)xanthine (E9)

Compound E9 was prepared from compound D5 by an analogous procedure to that of Example 7.

Melting point: 82°C

Elemental analysis

| | Calculated | Found |
|---|---|---|
| C | 57.13 | 57.42 |
| H | 7.19 | 7.16 |
| N | 16.66 | 16.73 |
| O | 19.02 | 18.86 |

Example 10

1-n-Butyl-3-(3-oxobutyl)-7-(2-hydroxypropyl)xanthine (E10)

Compound E10 was prepared from compound D8 (as unpurified raw material) by an analogous procedure to that of Example 1.

Melting point: 100°C.

Elemental analysis

| | Calculated | Found |
|---|---|---|
| C | 57.13 | 57.21 |
| H | 7.19 | 7.08 |
| N | 16.66 | 16.55 |
| O | 19.02 | 19.05 |

Example 11

1,3-bis-(3-Hydroxybutyl)-7-(2-oxopropyl)xanthine (E11)

Using an analogous procedure to that of Example 7, compound E11 was prepared from D10.

Melting point: 75° to 80°C (isolated as an oil which crystallised in the course of some months).

Example 12

1-(4-[2-Methyl-1,3-dioxolanyl-(2)]butyl)-3-(3-oxobutyl)-7-(3-[2-methyl-1,3-dioxolanyl(2)]propyl)xanthine (E12)

Compound E12 was prepared from compound D15 by an analogous procedure to that of Example 1.

Melting point: oil (tlc pure).

Example 13

1,3-bis-(3-Oxobutyl)-7-(2-oxopropyl)-xanthine(E13)

Compound E13 was prepared from compound E12 by an analogous procedure to that of Example 3.

Melting point: 151°C

Example 14

1,3-bis-(3-Hydroxybutyl)-7-(2-hydroxypropyl)xanthine (E14).

Compound E14 was prepared from compound E13 by an analogous procedure to that of Example 5.

Melting point: 88° to 97°C (isolated as an oil which crystallised in the course of some months).

Compound E14 is alternatively prepared from compound E11 by an analogous procedure to that of Example 5.

Example 15

1-(3,4-Dihydroxybutyl)-3-n-butyl-7-(2-hydroxypropyl)xanthine (E15)

Compound D16 (1g) was completely dissolved in methanol (16ml), and sodium borohydride (0.3g) was added in portions at room temperature. After about 30 minutes the reaction was complete (controlled by tlc).

To the reaction mixture ethanol (10ml), water (10ml) and concentrated hydrochloric acid (1ml) were added with stirring at room temperature. After a period of about 2 hours the reaction was complete.

Water (20ml) was added and the reaction mixture neutralised with diluted sodium hydroxide and extracted seven times with portions of methylene chloride (30ml). The methylene chloride phase was dried over sodium sulphate, filtered and the methylene chloride removed in vacuo.

Overnight the residue solidified and was treated with ether to yield 0.5g of a solid, melting point: 133°C. The structures of the compounds of Examples 1 to 11 and 13 to 15 were confirmed by NMR spectroscopy.

Pharmacological Data

1. Triethyltin-induced cerebral oedema in the rat.

The cerebral oedema is induced by oral administrations repeated for 5 consecutive days - one administration per day - or triethyltin chloride at a dose of 2 mg/kg. The study substances are also administered orally twice daily as aqueous solution or suspension at a dose of 1ml/100g body-weight; these administrations are given during the 5 days of intoxication with tin. Three groups of 10 male specific pathogen-free (SPF) Wistar rats of 280 ± 10g body-weight are used for each compound studied:

- 1 control group
- 1 group intoxicated with triethyltin
- 1 group intoxicated with triethyltin and treated with the studied compound.

The rats are killed on the evening of the fifth day; the brain is removed, weighed fresh and after desiccation to constant weight and the water content of each brain is calculated:

$[H_2O]$ = fresh weight - dry weight.

The following are then calculated:

- the mean water content (M ± Sm%) of each group;
- the protection index P due to the administered compound:

$$P\% = 1 - \frac{[H_2O]\text{ treated group} - [H_2O]\text{ control group}}{[H_2O]\text{triethyltin group} - [H_2O]\text{ control group}} \times 100$$

The results are shown in Table 1

TABLE 1

| Compound | Dosage mg/kg | % Reduction in cerebral oedema formation (P) |
|---|---|---|
| E5 | 2 x 30<br>2 x 100 | 40 *<br>100 * |
| E6 | 2 x 100 | 80 * |
| E7 | 2 x 20 | 89 ** |
| E8 | 2 x 50 | 89 ** |
| E9 | 2 x 12.5 | 100 ** |

* significant (p<0.05, Student's t-test)

**significant (p<0.01, signed rank test)

2. The Gerbil Ischaemic Deficit Passive Avoidance Test.

Mongolian gerbils were conditioned to avoid entering a dark compartment by means of a footshock (maximally 50V, 2s duration) received when entering from the light section of a two compartment box. Recollection of the footshock was examined 24h later by replacing the gerbils in the two compartment box and measuring the recall latency, the time taken to re-enter the dark compartment.

Effect of test compound on recall latency in the gerbil following transient forebrain ischaemia.

(a) Animal Preparation

A learning or memory deficit was induced in the gerbils by a transient (5min) bilateral carotid artery ligation, performed 24h prior to conditioning, under light hexobarbital anaesthesia.

(b) Measurement

Compounds, being examined for an effect on learning or memory in gerbils which had undergone carotid occlusion, were administered seven times during the experiment. The initial administration was during the period of forebrain ischaemia, the third and seventh administrations were 10min prior to conditioning and recall testing, respectively, and the remainder were given at intermediate time points.

Results were expressed as percentage of animals which had a long recall latency (>60s). A long recall latency indicates good information acquisition or retrival.

(c) Results

The results for test compounds are shown in Table 2.

TABLE 2

| | Percentage of animals with recall latencies >60s | |
|---|---|---|
| I | sham-ligated controls | 33 |
| II | Ischaemic controls | 14** |
| III | Ischaemia and Compound E5 (3mg/kg p.o.) | 32* |
| IV | Ischaemic and Compound E6 (3mg/kg p.o.) | 33* |

*significantly different from II ($p<0.05$).

**significantly different from I ($p<0.05$).

As can be seen in Table 1, transient cerebral ischaemia impairs the recollection of the footstock in gerbils. The test compounds significantly increased the percentage of animals with long recall latencies.

The above results show that the test compounds improve data acquisition or retrieval in the gerbil following transient forebrain ischaemia and demonstrate that the compounds of the invention are of potential use in the treatment of cerebral vascular and neuronal degenerative disorders associated with learning, memory and cognitive dysfunctions including cerebral senility, multi-infarct dementia and senile dementia of the Alzheimer type.

3. The effect of test compounds on the oxygen tension ($pO_2$) of rat ischaemic gastrocnemius muscle.

(a) Animal preparation

Male rats (325 to 390g), in which one femoral artery had been ligated 2-4 days previously, were were anaesthetized by i.p. injection of sodium-thiopentone (100 mg/kg).

(b) Measurement

The $pO_2$ of the gastrocnemius muscle of the ischaemic hindlimb was measured with surface electrodes. An incision was made through the skin on the inside of the ischaemic limb. The thick fascia covering the muscle surface was removed carefully and the surface electrode was placed on the gastrocnemius muscle.

The electrode current was measured every 6 to 8 s and collected for perlods of 4 min (Hewlett-Packard programmable data logger system 3051A). After each period, mean value and standard deviation was calculated. Muscle temperature was controlled by means of a thermocouple (Ellab, Copenhagen).

Upon a stable $pO_2$ measurement being obtained, test compound was administered intraduodenally via a plastic catheter.

The change in the muscle $pO_2$, produced by test compound administration, was calculated for each animal.

The results are shown in Table 3.

TABLE 3

| TEST COMPOUND | DOSAGE mg/kg i.v. | n | CHANGE in $pO_2$ (Torr) (mean ± SEM) |
|---|---|---|---|
| E5 | 5.0 | 4 | 2.3 ± 1.6* |
| E6 | 1.0 | 8 | 2.2 ± 0.6* |

i.v. intravenous adminstration

* Significance: $p<0.01$ (signed rank test)

4. Inhibition of cyclic AMP phosphodiesterase.

The activity of cyclic AMP phosphodiesterase in homogenates of rat skeletal muscle was measured in the presence of various concentrations of compounds of formula (I) using 0.31 μM cyclic AMP as substrate. The reciprocal of the reaction rate was plotted on the y axis against the concentration of each compound on the x axis (Dixon plot). The apparent Ki value was estimated by extrapolating the steeper linear portion of the Dixon plot to the x axis and obtaining the negative intercept on the x axis.

The results are shown in Table 4

TABLE 4

| Compound | Apparent Ki value (μ molar) |
|---|---|
| E5 | 128 |
| E6 | 94 |
| E7 | 13 |
| E8 | 160 |
| E9 | 13 |
| E11 | 81 |
| E14 | 815 |

The compounds of Examples 1 to 15 have the structures shown in Table 5:

TABLE 5

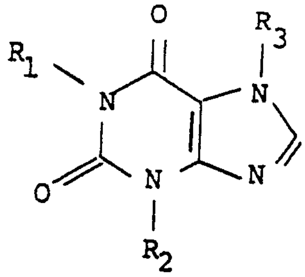

| Example | $R_1$ | $R_2$ | $R_3$ |
|---|---|---|---|
| 1 | $-(CH_2)_2COCH_3$ | $-nC_4H_9$ | 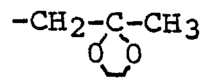 |
| 2 | $-nC_4H_9$ | $-(CH2)_2COCH_3$ | 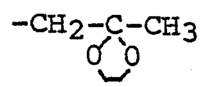 |
| 3 | $-(CH_2)_2COCH_3$ | $-nC_4H_9$ | $-CH_2COCH_3$ |
| 4 | $-nC_4H_9$ | $-(CH_2)_2COCH_3$ | $-CH_2COCH_3$ |
| 5 | $-(CH_2)_2CHOHCH_3$ | $-nC_4H_9$ | $-CH_2CHOHCH_3$ |
| 6 | $-nC_4H_9$ | $-(CH_2)_2CHOHCH_3$ | $-CH_2CHOHCH_3$ |
| 7 | $-(CH_2)_2CHOHCH_3$ | $-nC_4H_9$ | $-CH_2COCH_3$ |
| 8 | $-(CH_2)_2COCH_3$ | $-nC_4H_9$ | $-CH_2CHOHCH_3$ |
| 9 | $-nC_4H_9$ | $-(CH_2)_2CHOHCH_3$ | $-CH_2COCH_3$ |
| 10 | $-nC_4H_9$ | $-(CH_2)_2COCH_3$ | $-CH_2CHOHCH_3$ |
| 11 | $-(CH_2)_2CHOHCH_3$ | $-(CH_2)_2CHOHCH_3$ | $-CH_2COCH_3$ |
| 12 | $-(CH_2)_2-C-CH_3$ 0 0 | $-(CH_2)_2COCH_3$ | 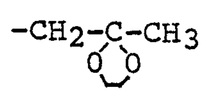 |
| 13 | $-(CH_2)_2CHOCH_3$ | $-(CH_2)_2COCH_3$ | $-CH_2COCH_3$ |
| 14 | $-(CH_2)_2CHOHCH_3$ | $-(CH_2)_2CHOHCH_3$ | $-CH_2CHOHCH_3$ |
| 15 | $-(CH_2)_2CHOHCH_2OH$ | $-nC_4H_9$ | $-CH_2CHOHCH3$ |

## Claims

1. A compound of formula (I):

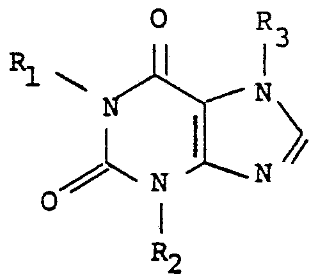

(I)

or a pharmaceutically acceptable salt thereof, wherein $R_1$ and $R_2$ are independently n-butyl, $-(CH_2)_2CHOHCH_3$, $-(CH_2)_2CHOHCH_2OH$, $-(CH_2)_2COCH_3$ or $-(CH_2)_2C(OR_4)(OR_5)CH_3$ where $R_4$ and $R_5$ are each $C_{1-4}$ alkyl or together are $C_{2-4}$ polymethylene, provided that $R_1$ and $R_2$ are not both n-butyl, and $R_3$ is $-CH_2CHOHCH_3$, $-CH_2COCH_3$ or $-CH_2C(OR_6)(OR_7)CH_3$ where $R_6$ and $R_7$ are each $C_{1-4}$ alkyl or together are $C_{2-4}$ polymethylene.

2. A compound according to claim 1, in which $R_1$ and $R_2$ are selected from n-butyl,

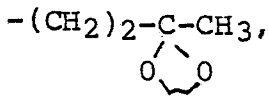

$-(CH_2)_2CHOHCH_2OH$, $-(CH_2)_2CHOHCH_3$ and $-(CH_2)_2COCH_3$.

3. A compound according to claim 1 or 2, in which $R_3$ is selected from $-CH_2CHOHCH_3$, $-CH_2COCH_3$ and

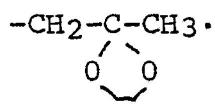

4. 2-Methyl-2-[(1-(3-oxobutyl)-3-n-butylxanthin-7-yl) methyl]-1,3-dioxolane,

2-methyl-2-[(1-n-butyl-3-(3-oxobutyl)xanthin-7-yl) methyl]-1,3-dioxolane,

1-(3-oxobutyl)-3-n-butyl-7-(2-oxopropyl)xanthine,

1-n-butyl-3-(3-oxobutyl)-7-(2-oxopropyl)xanthine,

1-(3-hydroxybutyl)-3-n-butyl-7-(2-hydroxypropyl) xanthine,

1-n-butyl-3-(3-hydroxybutyl)-7-(2-hydroxypropyl)xanthine,

1-(3-hydroxybutyl)-3-n-butyl-7-(2-oxopropyl)xanthine,

1-(3-oxobutyl)-3-n-butyl-7-(2-hydroxypropyl) xanthine,

1-n-butyl-3-(3-hydroxybutyl)-7-(2-oxopropyl)xanthine,

1-n-butyl-3-(3-oxobutyl)-7-(2-hydroxypropyl)xanthine,

1,3-bis-(3-hydroxybutyl)-7-(2-oxopropyl)xanthine,

1-(4-[2-methyl-1,3-dioxolanyl-(2)]butyl)-3-(3-oxobutyl)-7-(3-[2-methyl-1,3-dioxolanyl(2)]propyl)xanthine,

1,3-bis-(3-oxobutyl)-7-(2-oxopropyl)xanthine,

1,3-bis-(3-hydroxybutyl)-7-(2-hydroxypropyl)xanthine or 1-(3,4-dihydroxybutyl)-3-n-butyl-7-(2-hydroxypropyl) xanthine.

5. A process for the preparation of a compound according to claim 1, which process comprises reacting xanthine in which one of the 1 and 3 positions is substituted by $R_1'$ or $R_2'$, respectively, sequentially and in any appropriate order with compounds $R_3'X_3$ and $R_1'X_1$ or $R_2'X_2$ as appropriate, in which $R_1'$, $R_2'$ and $R_3'$ are $R_1$, $R_2$ and $R_3$ respectively, or groups convertible thereto and $X_1$, $X_2$ and $X_3$ are leaving groups, optionally with protection of xanthine nitrogen atoms, and at any appropriate stage, as necessary, converting $R_1'$, $R_2'$ and $R_3'$ to $R_1$, $R_2$ and $R_3$, interconverting $R_1$, $R_2$ and $R_3$ and optionally forming a pharmaceutically acceptable salt of the resulting compound of formula (I).

6. A pharmaceutical composition comprising a compound according to claim 1 and a pharmaceutically acceptable carrier.

7. A compound according to claim 1 for use as an active therapeutic substance.

8. A compound according to claim 1 for use in the treatment cerebral vascular and neuronal degenerative disorders associated with learning, memory and cognitive dysfunctions including cerebral senility, multi-infarct dementia and senile dementia of the Alzheimer type and/or peripheral vascular disease and/or proliferative skin diseases.

9. The use of a compound according to claim 1 for the preparation of a medicament for the treatment of cerebral vascular and neuronal degenerative disorders associated with learning, memory and cognitive dysfunctions including cerebral senility, multi-infarct dementia and senile dementia of the Alzheimer type and/or peripheral vascular disease and/or proliferative skin diseases.